# EUROPÄISCHE PATENTSCHRIFT

(11) **EP 3 755 203 B1**
(45) Veröffentlichungstag und Bekanntmachung des Hinweises auf die Patenterteilung: **10.01.2024**
(21) Anmeldenummer: 19706947.9
(22) Anmeldetag: 19.02.2019
(51) Int. Cl.: A61B 3/11

(54) **PUPILLOMETER UND VERFAHREN ZUM BETREIBEN DES PUPILLOMETERS**
PUPILLOMETER AND METHOD FOR OPERATING THE PUPILLOMETER
PUPILLOMÈTRE ET PROCÉDÉ DESTINÉ À FAIRE FONCTIONNER UN PUPILLOMÈTRE

(30) Priorität: 21.02.2018 DE 102018103867
(43) Veröffentlichungstag der Anmeldung: 30.12.2020
(73) Patentinhaber: Stellar Data Broadcast Services GmbH, 50354 Hürth (DE)
(72) Erfinder: JUNGGEBURTH, Johannes, 52372 Kreuzau (DE)
(74) Vertreter: PATERIS Patentanwälte PartmbB
(86) Internationale Anmeldenummer: PCT/EP2019/054057
(87) Internationale Veröffentlichungsnummer: WO 2019/162255

(56) Entgegenhaltungen:
- WO-A1-2015/120438
- US-A1- 2014 313 488
- US-A1- 2017 000 338

## Beschreibung

Die Erfindung betrifft ein Pupillometer und ein Verfahren zum Betreiben des Pupillometers.

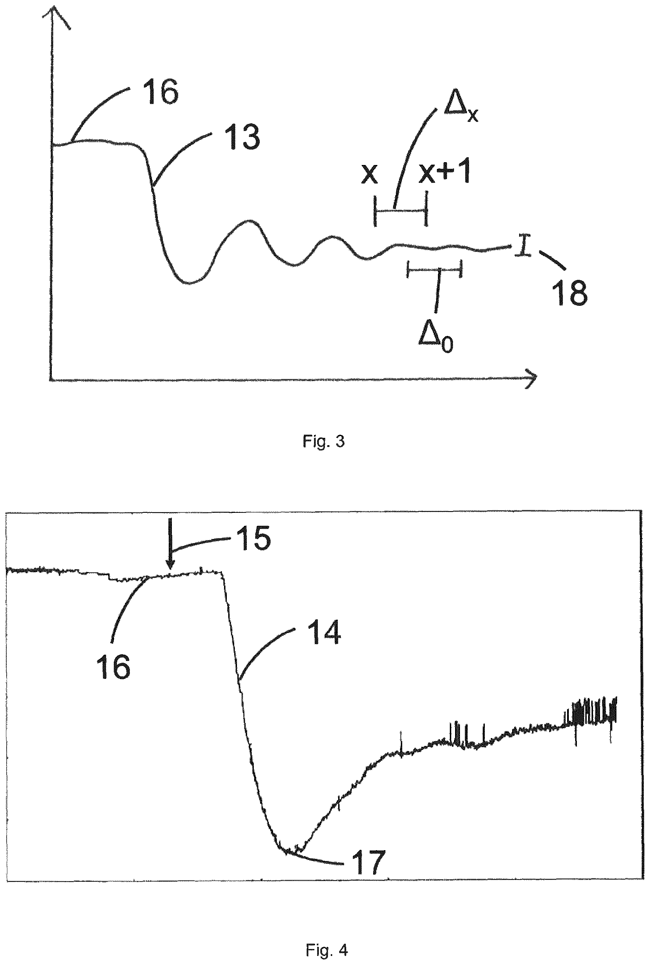

Ein Pupillenreflex bei einem Menschen führt dazu, dass, wenn der Mensch einer höheren Bestrahlung durch sichtbares Licht ausgesetzt ist, dies zu einer Verkleinerung einer Pupille des Menschen führt. Dadurch wird eine Lichtmenge verringert, die durch die Pupille auf die Netzhaut des Menschen fällt. Umgekehrt führt der Pupillenreflex bei einer niedrigeren Bestrahlung durch das sichtbare Licht dazu, dass die Pupille vergrö-ßert wird, wodurch eine größere Lichtmenge auf die Netzhaut fällt. In Figur 4 ist der Fall dargestellt, dass der Mensch mit einem Lichtpuls 15 angestrahlt wird, wie beispielsweise von einem Blitzlicht einer Kamera. Figur 4 zeigt ein Diagramm, in dem ein mit einem Pupillometer aufgenommener Pupillendurchmesserverlauf 14 dargestellt ist, wobei über eine Abszisse des Diagramms die Zeit und über eine Ordinate des Diagramms ein Pupillendurchmesser der Pupille aufgetragen ist. Man kann beobachten, dass sich zunächst die Pupille ausgehend von einem Ausgangspupillendurchmesser 16 bis zu einem Minimum 17 verkleinert. Anschließend vergrößert sich die Pupille wieder.

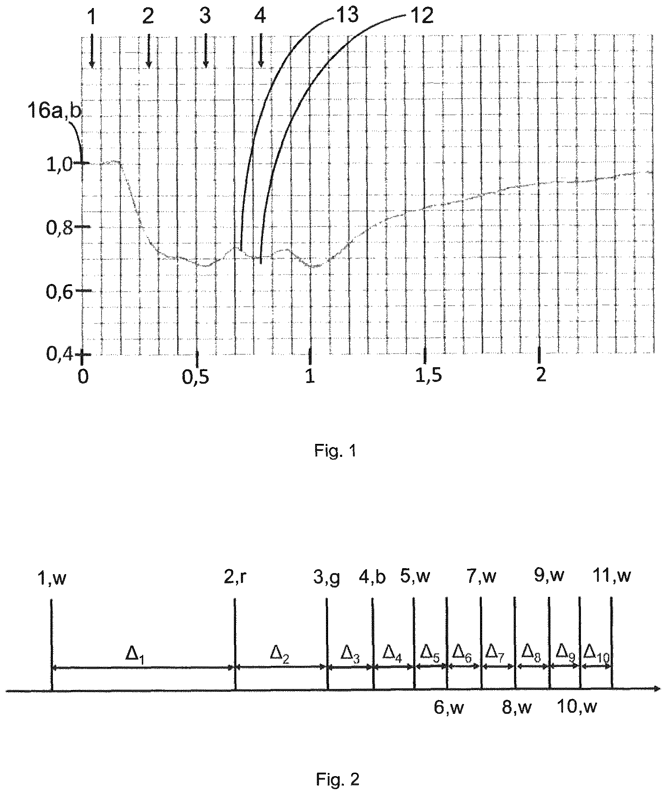

Es ist bekannt, dass man anhand des in Figur 1 dargestellten Pupillendurchmesserverlaufs auf eine körperliche Verfassung des Menschen zurückschließen kann. Bei der körperlichen Verfassung kann es sich beispielsweise um einen Grad von Müdigkeit oder um einen Blutalkoholgehalt des Menschen handeln. Nachteilig ist jedoch, dass aus dem in Figur 1 beispielhaft dargestellten Pupillendurchmesserverlauf nur eine geringe Menge an Informationen ausgelesen werden kann, so dass ein Rückschluss auf die körperliche Verfassung des Menschen nur ungenau ist.

US 2017/000338 A1 und US 2014/313488 A1 offenbaren Pupillometer.

Aufgabe der Erfindung ist es daher, eine Vorrichtung und ein Verfahren zu schaffen, die einen Pupillendurchmesserverlauf liefern, aus dem mit einer hohen Genauigkeit auf eine körperliche Verfassung eines Menschen zurückgeschlossen werden kann.

Das erste erfindungsgemäße Pupillometer ist im Anspruch 1 definiert und weist eine Lichtquelle, die eingerichtet ist, einen Menschen mit einer Pulssequenz zu beleuchten, so dass ein Pupillenreflex bei dem Menschen hervorgerufen wird, eine Kamera, die eingerichtet ist, eine Bildsequenz mit Bildern eines Auges des Menschen aufzunehmen, das eine Pupille mit dem Pupillenreflex zeigt, und eine Signalauswerteeinheit auf, die eingerichtet ist, in den Bildern einen Pupillendurchmesser und daraus einen Pupillendurchmesserverlauf zu bestimmen, wobei die Pulssequenz eine Mehrzahl von zeitlich aufeinanderfolgenden Lichtpulsen aufweist und zeitliche Abstände zwischen zwei benachbarten der Lichtpulse derart kurz sind, dass der Pupillendurchmesser während der Pulssequenz sich nicht auf einen Ausgangspupillendurchmesser erholt, der am Zeitpunkt des ersten Lichtpulses der Pulssequenz vorliegt, und bei der mindestens zwei der Lichtpulse eine unterschiedliche Farbe haben.

Das zweite erfindungsgemäße Pupillometer ist im Anspruch 2 definiert und weist eine Lichtquelle, die eingerichtet ist, einen Menschen mit einer Pulssequenz zu beleuchten, so dass ein Pupillenreflex bei dem Menschen hervorgerufen wird, eine Kamera, die eingerichtet ist, eine Bildsequenz mit Bildern eines Auges des Menschen aufzunehmen, das eine Pupille mit dem Pupillenreflex zeigt, und eine Signalauswerteeinheit auf, die eingerichtet ist, in den Bildern einen Pupillendurchmesser und daraus einen Pupillendurchmesserverlauf zu bestimmen, wobei die Pulssequenz eine Mehrzahl von zeitlich aufeinanderfolgenden Lichtpulsen aufweist und zeitliche Abstände zwischen zwei benachbarten der Lichtpulse derart kurz sind, dass der Pupillendurchmesser während der Pulssequenz sich nicht auf einen Ausgangspupillendurchmesser erholt, der am Zeitpunkt des ersten Lichtpulses der Pulssequenz vorliegt, wobei die Lichtquelle eingerichtet ist, den Menschen mit zwei der Pulssequenzen zu beleuchten, deren zeitlicher Pulssequenzabstand derart lang ist, dass sich der Pupillendurchmesser auf den Ausgangspupillendurchmesser erholt, wobei in einer der zwei der Pulssequenzen ein erster zeitlicher Abstand zwischen dem ersten Lichtpuls und einem späteren Lichtpuls derart ist, dass der spätere Lichtpuls während einer Pupillenkontraktion erfolgt, und in einer anderen der zwei der Pulssequenzen ein zweiter zeitlicher Abstand zwischen dem ersten Lichtpuls und einem späteren Lichtpuls, der dem späteren Lichtpuls in der einen der zwei der Pulssequenzen entspricht, derart ist, dass der spätere Lichtpuls während einer Pupillendilatation erfolgt, wobei das Pupillometer eingerichtet ist, eine erste Anfangsbeschleunigung in dem Pupillendurchmesserverlauf nach dem späteren Lichtpuls der einen der zwei Pulssequenzen und eine zweite Anfangsbeschleunigung in dem Pupillendurchmesserverlauf nach dem späteren Lichtpuls der anderen der zwei der Pulssequenzen zu bestimmen.

Das erfindungsgemäße Verfahren zum Betreiben des Pupillometers ist im Anspruch 13 definiert und weist die Schritte auf: a) Beleuchten des Menschen mit einer Lichtquelle, so dass ein Pupillenreflex bei dem Menschen hervorgerufen wird, mit einer Pulssequenz, die eine Mehrzahl von zeitlich aufeinanderfolgenden Lichtpulsen aufweist, wobei zeitliche Abstände zwischen zwei benachbarten der Lichtpulse derart kurz sind, dass der Pupillendurchmesser während der Pulssequenz sich nicht auf einen Ausgangspupillendurchmesser erholt, der am Zeitpunkt des ersten Lichtpulses der Pulssequenz vorliegt, und bei der mindestens zwei der Lichtpulse eine unterschiedliche Farbe haben; b) Aufnehmen einer Bildsequenz mit Bildern eines Auges des Menschen mit der Kamera, wobei die Pupille des Auges den Pupillenreflex zeigt; c) Bestimmen in den Bildern einen Pupillendurchmesser und daraus einen Pupillendurchmesserverlauf.

Indem nun der Mensch mit der Pulssequenz und gemäß dem ersten erfindungsgemä-ßen Pupillometer mit den unterschiedlichen Farben bestrahlt wird, hat dadurch der Pupillendurchmesserverlauf eine höhere Komplexität als ein herkömmlicher Pupillendurchmesserverlauf, bei dem lediglich ein einziger Lichtpuls eingesetzt wird. Beispielsweise zeichnet sich der Pupillendurchmesserverlauf dadurch aus, dass er mehrere lokale Minima und mehrere lokale Maxima haben kann. Weil die unterschiedlichen Farben unterschiedliche Pupillenreflexe auslösen, ist die Komplexität des Pupillendurchmesserverlaufs besonders hoch. Durch diese besonders hohe Komplexität können in dem Pupillendurchmesserverlauf, der mit dem erfindungsgemäßen Pupillometer oder mit dem erfindungsgemäßen Verfahren bestimmt wird, mehr Informationen als in dem herkömmlichen Pupillendurchmesserverlauf herausgelesen werden. Dadurch kann mit einer höheren Genauigkeit auf die körperliche Verfassung des Menschen zurückgeschlossen werden.

Wie genau der Pupillendurchmesserverlauf beschaffen ist, variiert von Mensch zu Mensch. Durch die besonders hohe Komplexität des Pupillendurchmesserverlaufs ist es möglich, einem Pupillendurchmesserverlauf einem Menschen zuzuordnen, ähnlich wie bei einem Fingerabdruck. Der Pupillendurchmesserverlauf ist dementsprechend stark personalisiert. Mit dem erfindungsgemäßen Pupillometer und dem erfindungsgemäßen Verfahren ist es somit möglich, einen Arbeitsplatz personalisiert abzusichern. Beispielsweise kann der Pupillendurchmesserverlauf als ein Referenzpupillendurchmesserverlauf bei dem Menschen bestimmt werden, wenn er bei einer guten körperlichen Verfassung ist. Dies kann beispielsweise bedeuten, dass er gesund ist und keinerlei psychoaktiven Substanzen, wie beispielsweise Alkohol oder Psychopharmaka, in seinem Blutkreislauf hat. Zu einem Absichern des Arbeitsplatzes kann zu einem späteren Zeitpunkt ein erneuter Pupillendurchmesserverlauf des Menschen bestimmt werden. Durch die hohe Komplexität des Pupillendurchmesserverlaufs können selbst kleinste Abweichungen des erneuten Pupillendurchmesserverlaufs zu dem Referenzpupillendurchmesserverlauf erkannt werden. Aus den Abweichungen kann dann mit einer besonders hohen Genauigkeit auf die körperliche Verfassung des Menschen zurückgeschlossen werden. Beispielsweise kann dies in der Bestimmung eines Blutalkoholgehalts oder in einem Hinweis auf eine neuronale Erkrankung liegen. Auch ist es aufgrund des stark personalisierten Pupillendurchmesserverlaufs möglich zu erkennen, ob der erneute Pupillendurchmesserverlauf tatsächlich von dem gleichen Menschen wie bei dem Referenzpupillendurchmesser aufgenommen wurde. Sollte bei dem Menschen dann eine schlechte körperliche Verfassung vorliegen, kann zu der Absicherung des Arbeitsplatzes dem Menschen der Zugang zu diesem verwehrt werden.

Auch ist es aufgrund der hohen Komplexität möglich, durch einen Vergleich des Pupillendurchmesserverlaufs mit Pupillendurchmesserverläufen, die an einer Testgruppe von Menschen aufgenommen wurden, das Alter des Menschen mit einer hohen Genauigkeit zu bestimmen.

Zwei Muskeln, die beide von dem Gehirn des Menschen angesteuert werden, steuern den Pupillendurchmesser. Dadurch, dass bei dem zweiten erfindungsgemäßen Pupillometer während der Pupillenkontraktion und während der Pupillendilatation der Mensch beleuchtet wird, ergeben sich unterschiedliche Pupillendurchmesserverläufe. Durch das Beleuchten während der Pupillenkontraktion wird die Pupillenkontraktion noch weiter beschleunigt. Durch das Beleuchten während der Pupillenkontraktion wird gegen den Muskel gearbeitet. Dies resultiert in einer Differenz zwischen der ersten Anfangsbeschleunigung und der zweiten Anfangsbeschleunigung. Aus dieser Differenz kann beispielsweise auf eine mögliche Erkrankung des Menschen zurückgeschlossen werden, wobei die Erkrankung altersbedingt sein kann.

Es ist wesentlich, dass die Lichtpulse in der Pulssequenz des Pupillometers nach Anspruch 1 zeitlich derart angeordnet sind, dass die zeitlichen Abstände in Richtung fortschreitender Zeit immer kürzer werden. Es ist bevorzugt, dass die Lichtpulse in der Pulssequenz des Pupillometers nach Anspruch 2 zeitlich derart angeordnet sind, dass die zeitlichen Abstände in Richtung fortschreitender Zeit immer kürzer werden. Dadurch wird der Pupillendurchmesserverlauf noch komplexer. Weil unterschiedliche Menschen unterschiedlich schnell auf die Lichtpulse reagieren, wird der Pupillendurchmesserverlauf noch stärker personalisiert.

Das Pupillometer nach Anspruch 1 ist eingerichtet, einen zeitlichen Grenzabstand zwischen zwei benachbarten der Lichtpulse zu bestimmen, bei dem die Pupille ihren Pupillendurchmesser nicht mehr verändert. Das Pupillometer nach Anspruch 2 ist bevorzugt eingerichtet, einen zeitlichen Grenzabstand zwischen zwei benachbarten der Lichtpulse zu bestimmen, bei dem die Pupille ihren Pupillendurchmesser nicht mehr verändert. Der zeitliche Grenzabstand ist eine weitere Information, die sich aus dem Pupillendurchmesserverlauf herauslesen lässt. Durch diese weitere Information kann mit einer noch höheren Genauigkeit auf die körperliche Verfassung des Menschen zurückgeschlossen werden. Weil der zeitliche Grenzabstand bei unterschiedlichen Menschen auch unterschiedlich ist, ist der Pupillendurchmesserverlauf zudem noch stärker personalisiert. Es ist bevorzugt, dass das Pupillometer eingerichtet ist, den Grenzabstand zu bestimmen, indem in der Richtung fortschreitender Zeit für jeweils zwei benachbarte der Lichtpulse in dem Pupillendurchmesserverlauf geprüft wird, ob der Pupillendurchmesser in einem zeitlichen Prüfintervall innerhalb eines Toleranzbereichs liegt, insbesondere wobei der Toleranzbereich maximal 10 %, insbesondere maximal 5 %, des Ausgangspupillendurchmessers beträgt. Dabei handelt es sich um einfache und damit schnell durchführbare Rechenschritte, so dass der Grenzabstand schnell bestimmbar ist. Das zeitliche Prüfintervall ist bevorzugt mindestens so lang wie der zeitliche Abstand zwischen den jeweiligen zwei benachbarten der Lichtpulse und liegt bevorzugt zeitlich nach mindestens einem der jeweiligen zwei der benachbarten Lichtpulse. Damit kann der Grenzabstand mit einer besonders hohen Genauigkeit bestimmt werden.

Es ist bevorzugt, dass die zeitlichen Abstände maximal 1,00 s sind, und insbesondere minimal 0,10 s sind. Mit diesen zeitlichen Abständen kann der Pupillendurchmesserverlauf vorteilhaft in einem kurzen Zeitraum mit einer gleichzeitig sehr hohen Komplexität bestimmt werden. Der zeitliche Abstand ist dabei der Abstand der zeitlichen Mittelpunkte der zwei benachbarten der Lichtpulse.

Es ist bevorzugt, dass die Lichtquelle eingerichtet ist, lediglich eines der Augen des Menschen mit der Pulssequenz zu beleuchten, und die Kamera eingerichtet ist, die Bildsequenz mit den Bildern des anderen Auges des Menschen aufzunehmen, insbesondere ist das Pupillometer eingerichtet, jeweils eine Bildsequenz mit Bildern von jedem der Augen des Menschen aufzunehmen, und insbesondere ist die Signalauswerteeinheit eingerichtet, für jedes der Augen einen jeweiligen Pupillendurchmesserverlauf zu bestimmen. Ein Nervensignal, das von lediglich dem einen Auge erzeugt wird, muss einmal durch das Stammhirn des Menschen geleitet werden, bevor es auf dem anderen Auge ankommt und dort den Pupillenreflex auslöst. Dadurch wird das Stammhirn getestet, so dass der Pupillendurchmesserverlauf noch mehr Informationen enthält, aus denen auf die körperliche Verfassung des Menschen rückschließbar ist. Insbesondere kann dadurch, dass das Stammhirn getestet wird, auf eine neuronale Erkrankung des Menschen mit einer hohen Genauigkeit zurückgeschlossen werden. In dem Fall, dass von jedem der Augen der jeweilige Pupillendurchmesserverlauf bestimmt wird, kann das Pupillometer zwei der Kameras aufweisen, wobei eine der zwei Kameras eingerichtet ist, die Bildsequenz von dem einem Auge aufzunehmen, und die andere der zwei Kameras eingerichtet ist, die Bildsequenz von dem anderen Auge aufzunehmen. Indem von jedem der Augen der jeweilige Pupillendurchmesserverlauf bestimmt wird aber nur lediglich das eine Auge beleuchtet wird, lassen sich aus den beiden Pupillendurchmesserverläufen besonders viele Informationen herauslesen. Insbesondere lässt sich ein Vergleich der beiden Pupillendurchmesserverläufe vornehmen, aus dem weitere Informationen herauslesbar sind. Insbesondere kann aus einer langen Verzögerung des Pupillendurchmesserverlaufs des anderen Auges im Vergleich zu dem einen Auge mit einer hohen Genauigkeit auf eine neuronale Erkrankung, insbesondere des Stammhirns, zurückgeschlossen werden.

Die unterschiedlichen Farben weisen bevorzugt Rot, Grün, Blau und insbesondere Weiß auf.

Es ist bevorzugt, dass das Verfahren die Schritte aufweist: e) Speichern des Pupillendurchmesserverlaufs; f) Wiederholen der Schritte a) bis c) und Vergleichen des in Schritt f) bestimmten Pupillendurchmesserverlaufs mit dem in Schritt e) gespeicherten Pupillendurchmesserverlauf. Der in Schritt e) gespeicherte Pupillendurchmesserverlauf kann der Referenzpupillendurchmesserverlauf sein, der zu bestimmen ist, wenn der Mensch in der guten körperlichen Verfassung ist. Dies kann beispielsweise bedeuten, dass er gesund ist und keinerlei psychoaktiven Substanzen, wie beispielsweise Alkohol oder Psychopharmaka, in seinem Blutkreislauf hat. Durch den in Schritt f) durchgeführten Vergleich kann mit einer besonders hohen Genauigkeit auf die körperliche Verfassung des Menschen zurückgeschlossen werden.

Es ist bevorzugt, dass in Schritt a) die Lichtpulse in der Pulssequenz zeitlich derart angeordnet werden, dass die zeitlichen Abstände in Richtung fortschreitender Zeit immer kürzer werden; und das Verfahren den Schritt aufweist: d) Bestimmen eines zeitlichen Grenzabstands zwischen zwei benachbarten der Lichtpulse, bei dem die Pupille ihren Pupillendurchmesser nicht mehr verändert. In Schritt d) wird bevorzugt in der Richtung fortschreitender Zeit für jeweils zwei benachbarte der aufeinanderfolgenden Lichtpulse in dem Pupillendurchmesserverlauf geprüft, ob der Pupillendurchmesser in einem zeitlichen Prüfintervall innerhalb eines Toleranzbereichs liegt, insbesondere wobei der Toleranzbereich maximal 10 %, insbesondere maximal 5 %, des Ausgangspupillendurchmessers beträgt. Es ist bevorzugt, dass das zeitliche Prüfintervall mindestens so lang wie der zeitliche Abstand zwischen den jeweiligen zwei benachbarten der Lichtpulse ist und zeitlich nach mindestens einem der jeweiligen zwei der benachbarten Lichtpulse liegt.

Es ist zudem bevorzugt, dass die zeitlichen Abstände maximal 1,00 s sind, und insbesondere minimal 0,10 s sind.

Es ist bevorzugt, dass in Schritt a) lediglich eines der Augen des Menschen mit der Pulssequenz beleuchtet wird, und in Schritt b) die Bildsequenz mit den Bildern des anderen Auges des Menschen aufgenommen wird, insbesondere wird jeweils eine Bildsequenz mit Bildern von jedem der Augen des Menschen aufgenommen, und insbesondere wird in Schritt c) für jedes der Augen ein jeweiliger Pupillendurchmesserverlauf bestimmt.

Die unterschiedlichen Farben weisen bevorzugt Rot, Grün, Blau und insbesondere Weiß auf.

Das Pupillometer weist bevorzugt eine weitere Lichtquelle auf, die eingerichtet ist, das Auge vor jeder der Pulssequenzen mit Dauerlicht mit einer definierten Lichtstärke von sichtbarem Licht zu beleuchten. Besonders bevorzugt ist die definierte Lichtstärke ausgewählt aus einem Bereich von 5 lux bis 20 lux, insbesondere von 7 lux bis 14 lux.

Dadurch kann bei verschiedenen Pupillendurchmesserverläufen ein ähnlicherer Ausgangspupillendurchmesser erreicht werden als es der Fall ist, wenn das Auge sich im Dunkeln befindet. Dadurch können die Informationen wesentlich zuverlässiger aus den Pupillendurchmesserverläufen gelesen werden als es der Fall ist, wenn das Auge sich im Dunkeln befindet.

Im Folgenden wird anhand der beigefügten schematischen Zeichnungen die Erfindung näher erläutert.
Figur 1 zeigt zwei Pupillendurchmesserverläufe, die mit einer Pulssequenz aufgenommen wurden.
Figur 2 zeigt eine andere Pulssequenz.
Figur 3 zeigt einen Pupillendurchmesserverlauf, bei dem ein zeitlicher Grenzabstand ermittelt wurde.
Figur 4 zeigt einen herkömmlichen Pupillendurchmesserverlauf.
Figur 5 zeigt zwei Pupillendurchmesserverläufe, die mit zwei verschiedenen Pulssequenzen aufgenommen wurden.

Ein Pupillometer weist eine Lichtquelle, eine Kamera und eine Signalauswerteeinheit auf. Die Lichtquelle ist eingerichtet ist, einen Menschen mit einer Pulssequenz zu beleuchten, so dass ein Pupillenreflex bei dem Menschen hervorgerufen wird. Die Kamera ist eingerichtet, eine Bildsequenz mit Bildern eines Auges des Menschen aufzunehmen, das eine Pupille mit dem Pupillenreflex zeigt. Die Signalauswerteeinheit ist eingerichtet, in den Bildern einen Pupillendurchmesser und daraus einen Pupillendurchmesserverlauf zu bestimmen. Zum Bestimmen des Pupillendurchmessers kann ausgenutzt werden, dass die Pupille dunkler ist als der die Pupille umgebende Teil des Auges. Die Pupille des Auges kann somit in jedem der Bilder beispielsweise zwischen einem abrupten Abfall und einem abrupten Anstieg der Helligkeit lokalisiert werden und der Pupillendurchmesser kann als der Abstand zwischen dem abrupten Abfall und dem abrupten Anstieg bestimmt werden. Dies und weitere Details zu der Bestimmung des Pupillendurchmessers sind in DE 10 2016 104 417 A1 beschrieben.

Wie es in Figuren 1 und 2 dargestellt ist, weist die Pulssequenz eine Mehrzahl von zeitlich aufeinanderfolgenden Lichtpulsen 1 bis 11 auf. Zeitliche Abstände Δ₁ bis Δ₁₀ zwischen zwei benachbarten der Lichtpulse sind derart kurz, dass der Pupillendurchmesser während der Pulssequenz sich nicht auf einen Ausgangspupillendurchmesser 16 erholt, der am Zeitpunkt des ersten Lichtpulses 1 der Pulssequenz vorliegt. Der Ausgangspupillendurchmesser 16 kann bestimmt werden, indem der Pupillendurchmesser in einem Zeitbereich gemittelt wird, der vor einem Eintritt des Pupillenreflexes liegt. Der Zeitpunkt des ersten Lichtpulses 1 kann den Zeitbereich begrenzen oder innerhalb des Zeitbereichs liegen. Mindestens zwei der Lichtpulse haben eine unterschiedliche Farbe. Die unterschiedlichen Farben können Rot (r), Grün (g), Blau (b) und Weiß (w) aufweisen.

Beispielhaft kann das Pupillometer eine Brille aufweisen, die von dem Menschen aufzusetzen ist. Die Brille kann eine Kapsel aufweisen, die an das Gesicht des Menschen gedrückt eingerichtet ist, kein Umgebungslicht auf die Augen auftreffen zu lassen. Die Lichtquelle kann in der Brille untergebracht sein. Zudem kann die Lichtquelle eine rote LED, die eingerichtet ist rotes Licht zu emittieren, eine grüne LED, die eingerichtet ist grünes Licht zu emittieren, und eine blaue LED aufweisen, die eingerichtet ist blaues Licht zu emittieren. Dadurch können die unterschiedlichen Farben erzeugt werden. Durch Mischen des Lichts all der LEDs kann die Farbe Weiß erzeugt werden.

Die Lichtpulse können eine Dauer von kürzer als 10 ms, insbesondere kürzer als 1,5 ms (volle Halbwertsbreite, FWHM) haben. Zudem können die Lichtpulse länger als 0,1 ms (volle Halbwertsbreite, FWHM) sein. Die Beleuchtungsstärke auf dem Auge kann von 100 lux bis 200 lux betragen.

Zudem kann das Pupillometer eine Infrarotlichtquelle aufweisen, die eingerichtet ist, das Auge mit Infrarotlicht zu beleuchten, beispielsweise mit einer Wellenlänge von 940 nm. Bei der Infrarotlichtquelle kann es sich beispielsweise um eine infrarote LED handeln. Die Kamera ist eingerichtet, das Infrarotlicht zu detektieren. Die Kamera kann eingerichtet sein, die Bilder in der Bildsequenz mit einer Wiederholrate von 240 Hz bis 1000 Hz aufzunehmen.

Figur 1 zeigt eine Ausführungsform, bei der die Lichtquelle eingerichtet ist, lediglich eines der Augen des Menschen zu beleuchten. Dazu kann die Brille eine Trennwand aufweisen, die unterbindet, dass von der Lichtquelle Licht auf das andere Auge gelangt. Alternativ kann die Brille auch zwei der Kapseln, eine für jedes Auge, aufweisen. Das Pupillometer weist zwei der Kameras auf, wobei eine der zwei Kameras eingerichtet ist, die Bildsequenz mit den Bildern des einen Auges des Menschen aufzunehmen, und die andere der zwei Kameras eingerichtet ist, die Bildsequenz mit den Bildern des anderen Auges des Menschen aufzunehmen. Die Signalauswerteeinheit ist eingerichtet, für jedes der Augen einen jeweiligen Pupillendurchmesserverlauf 12, 13 zu bestimmen, wobei in Figur 1 ein Pupillendurchmesserverlauf 12 des anderen Auges und ein Pupillendurchmesserverlauf 13 des einen Auges dargestellt ist. Der Pupillendurchmesserverlauf 12 hat einen Ausgangspupillendurchmesser 16a und der Pupillendurchmesserverlauf 13 hat einen Ausgangspupillendurchmesser 16b. Für einen Vergleich des Pupillendurchmesserverlaufs 12 des anderen Auges mit dem Pupillendurchmesserverlauf 13 des einen Auges kann die Signalauswerteeinheit eingerichtet sein, eine Differenz zwischen dem Pupillendurchmesserverlauf 12 des anderen Auges und dem Pupillendurchmesserverlauf 13 des einen Auges zu bilden, so dass ein Differenzpupillendurchmesserverlauf gebildet wird. Die Pulssequenz der Ausführungsform gemäß Figur 1 hat vier der Lichtpulse, nämlich einen ersten Lichtpuls 1, der die Farbe Weiß hat, einen zweiten Lichtpuls 2, der die Farbe Rot hat, einen dritten Lichtpuls 3, der die Farbe Grün hat, und einen vierten Lichtpuls 4, der die Farbe Blau hat. Die Pupillendurchmesserverläufe 12, 13 weisen mehrere lokale Maxima und mehrere lokale Minima auf.

Figur 2 zeigt eine Ausführungsform für die Pulssequenz, die verschieden von der Pulssequenz gemäß Figur 1 ist. In der Pulssequenz gemäß Figur 2 sind die Lichtpulse 1 bis 11 zeitlich derart angeordnet, dass die zeitlichen Abstände Δ₁ bis Δ₁₀ in Richtung fortschreitender Zeit immer kürzer werden. Der zeitliche Abstand ist dabei der Abstand der zeitlichen Mittelpunkte der zwei benachbarten der Lichtpulse. Beispielsweise kann der zeitliche Abstand von 1,00 s bis 0,10 s, insbesondere von 1,00 s bis 0,15 s verkürzt werden. Beispielsweise können der erste Lichtpuls 1 die Farbe Weiß (w), der zweite Lichtpuls 2 die Farbe Rot (r), der dritte Lichtpuls 3 die Farbe Grün (g), der vierte Lichtpuls 4 die Farbe Blau (b) und alle folgenden Lichtpulse 5 bis 11 die Farbe Weiß (w) haben.

Beispielhaft hat die Pulssequenz gemäß Figur 2 elf Lichtpulse 1 bis 11, nämlich einen ersten Lichtpuls 1, der die Farbe Weiß (w) hat, einen zweiten Lichtpuls 2, der die Farbe Rot (r) hat, einen dritten Lichtpuls 3, der die Farbe Grün (g) hat, einen vierten Lichtpuls 4, der die Farbe Blau (b) hat, und einen fünften bis elften Lichtpuls 5 bis 11, die jeweils die Farbe Weiß (w) haben. Beispielhafte zeitliche Abstände Δ₁ bis Δ₁₀ und die zugehörigen Frequenzen sind in nachfolgender Tabelle angegeben:

| | Zeitlicher Abstand [s] | Frequenz [Hz] |
|---|---|---|
| Δ₁ | 1,00 | 1,00 |
| Δ₂ | 0,50 | 2,00 |
| Δ₃ | 0,250 | 4,00 |
| Δ₄ | 0,225 | 4,44 |
| Δ₅ | 0,200 | 5,00 |
| Δ₆ | 0,192 | 5,20 |
| Δ₇ | 0,185 | 5,40 |
| Δ₈ | 0,179 | 5,60 |
| Δ₉ | 0,172 | 5,80 |
| Δ₁₀ | 0,167 | 6,00 |

In Figur 3 ist dargestellt, wie anhand einer im Zusammenhang mit der Figur 2 beschriebenen Pulssequenz ein zeitlicher Grenzabstand Δₓ zwischen zwei benachbarten der Lichtpulse x und x+1 bestimmbar ist, bei dem die Pupille ihren Pupillendurchmesser nicht mehr verändert. Dazu ist das Pupillometer eingerichtet, in der Richtung fortschreitender Zeit für jeweils zwei benachbarte der Lichtpulse in dem Pupillendurchmesserverlauf 12, 13 zu prüfen, ob der Pupillendurchmesser in einem zeitlichen Prüfintervall Δ₀ innerhalb eines Toleranzbereichs 18 liegt. Diese Prüfung kann für alle die zwei benachbarten der Lichtpulse x und x+1 erfolgen. Alternativ kann die Prüfung erst erfolgen, wenn der zeitliche Abstand von den zwei benachbarten der Lichtpulsen x und x+1 kürzer oder gleich einer Schwelle ist, wie beispielsweise 0,25 s. Der Toleranzbereich 18 kann beispielsweise maximal 10 %, insbesondere maximal 5 %, des Ausgangspupillendurchmessers 16 betragen. Das zeitliche Prüfintervall Δ₀ ist mindestens so lang wie der zeitliche Abstand zwischen den jeweiligen zwei benachbarten der Lichtpulse x, x+1. In Figur 4 ist dargestellt, dass das Prüfintervall Δ₀ zwischen dem ersten Lichtpuls x der zwei benachbarten der Lichtpulse und dem zweiten Lichtpuls x+1 der zwei benachbarten der Lichtpulse liegt. Alternativ ist denkbar, dass das Prüfintervall Δ₀ nach dem zweiten Lichtpuls x+1 der zwei benachbartender Lichtpulse liegt. Zum Prüfen, ob der Pupillendurchmesser innerhalb des Toleranzbereichs 18 liegt, können in dem Prüfintervall Δ₀ ein maximaler Pupillendurchmesser dₘₐₓ und ein minimaler Pupillendurchmesser dₘᵢₙ bestimmt werden und geprüft werden, ob eine Differenz dₘₐₓ-dₘᵢₙ kleiner oder gleich dem Toleranzbereich 18 ist.

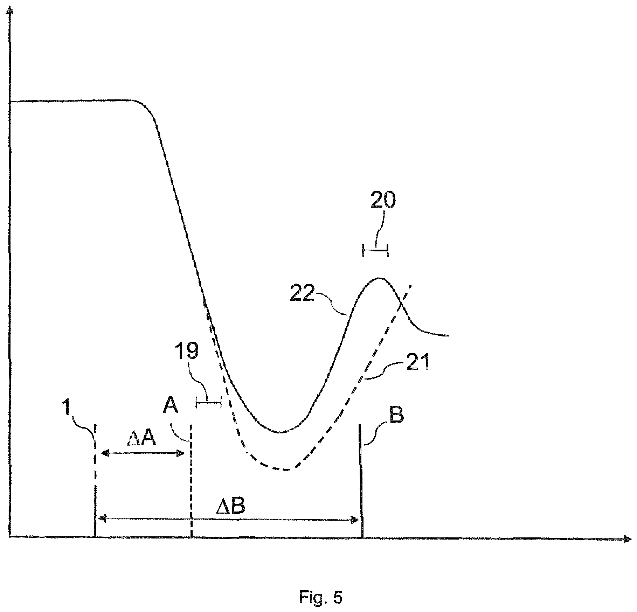

Figur 5 zeigt Pupillendurchmesserverläufe, die mit dem Pupillometer aufgenommen worden sind, wobei die Lichtquelle eingerichtet ist, den Menschen mit zwei der Pulssequenzen zu beleuchten, deren zeitlicher Pulssequenzabstand derart lang ist, dass sich der Pupillendurchmesser auf den Ausgangspupillendurchmesser 16 erholt. Um die Länge des zeitlichen Pulssequenzabstands zu bestimmen, können Experimente durchgeführt werden. Alternativ kann ein sehr langer zeitlicher Pulssequenzabstand gewählt werden, wie beispielsweise 15 s. In einer der zwei der Pulssequenzen, die in Figur 5 durch die vertikalen gestrichelten Linien dargestellt ist, ist ein erster zeitlicher Abstand Δ_{A} zwischen dem ersten Lichtpuls 1 und einem späteren Lichtpuls A derart, dass der spätere Lichtpuls A während einer Pupillenkontraktion erfolgt, und in einer anderen der zwei der Pulssequenzen, die in Figur 5 durch die vertikalen durchgezogenen Linien dargestellt ist, ein zweiter zeitlicher Abstand Δ_{B} zwischen dem ersten Lichtpuls 1 und einem späteren Lichtpuls B, der dem späteren Lichtpuls A in der einen der zwei der Pulssequenzen entspricht, derart ist, dass der spätere Lichtpuls B während einer Pupillendilatation erfolgt. In Figur 1 hat der Pupillendurchmesserverlauf, der zu der einen der zwei der Pulssequenzen (mit dem zeitlichen Abstands Δ_{A}) gehört, das Bezugszeichen 21 und der Pupillendurchmesserverlauf, der zu der anderen der zwei der Pulssequenzen Pulssequenzen (mit dem zeitlichen Abstands Δ_{B}) gehört, das Bezugszeichen 22. Der spätere Lichtpuls B entspricht dem späteren Lichtpuls A, wenn sie beide die gleiche Ordnungszahl in der Pulssequenz haben, d.h. der spätere Lichtpuls A und der spätere Lichtpuls B sind beide beispielsweise der zweite Lichtpuls 2 in der Pulssequenz oder der dritte Lichtpuls 3 in der Pulssequenz. Die zeitlichen Abstände Δ_{A} und Δ_{B} können beispielsweise von einem Bediener des Pupillometers durch einfaches Ausprobieren herausgefunden werden. Es kann erforderlich sein, für jeden Menschen ein neues Wertepaar Δ_{A} und Δ_{B} zu finden. Auch kann das Pupillometer eingerichtet sein, automatisch das Wertepaar Δ_{A} und Δ_{B} durch Ausprobieren finden.

Das Pupillometer ist eingerichtet, eine erste Anfangsbeschleunigung in dem Pupillendurchmesserverlauf 21 nach dem späteren Lichtpuls A der einen der zwei Pulssequenzen und eine zweite Anfangsbeschleunigung in dem Pupillendurchmesserverlauf 22 nach dem späteren Lichtpuls B der anderen der zwei der Pulssequenzen zu bestimmen. Die erste Anfangsbeschleunigung und die zweite Anfangsbeschleunigung sind dabei die zweite Ableitung des Pupillendurchmessers nach der Zeit. Beispielsweise kann die erste Anfangsbeschleunigung nach erster Dauer 19 nach dem späteren Lichtpuls A und die zweite Anfangsbeschleunigung kann nach einer zweiten Dauer 20 nach dem späteren Lichtpuls B aufgenommen werden. Dabei können die ersten Dauer 19 und die zweite Dauer 20 gleich lang sein. Auch ist es möglich, für die erste Anfangsbeschleunigung und die zweite Anfangsbeschleunigung einen Mittelwert der zweiten Ableitungen des Pupillendurchmessers nach der Zeit über die erste Dauer 19 beziehungsweise die zweite Dauer 20 zu bilden.

Zudem kann das Pupillometer eingerichtet sein, eine Differenz zwischen der ersten Anfangsbeschleunigung und der zweiten Anfangsbeschleunigung zu bilden.

### Bezugszeichenliste

1 erster Lichtpuls
2 zweiter Lichtpuls
3 dritter Lichtpuls
4 vierter Lichtpuls
5 fünfter Lichtpuls
6 sechster Lichtpuls
7 siebter Lichtpuls
8 achter Lichtpuls
9 neunter Lichtpuls
10 zehnter Lichtpuls
11 elfter Lichtpuls
12 Pupillendurchmesserverlauf des anderen Auges
13 Pupillendurchmesserverlauf des einen Auges
14 herkömmlicher Pupillendurchmesserverlauf
15 Lichtpuls
16 Ausgangspupillendurchmesser
16a Ausgangspupillendurchmesser des anderen Auges
16b Ausgangspupillendurchmesser des einen Auges
17 Minimum
18 Toleranzbereich
Δ₁ bis Δ₁₀ zeitlicher Abstand
Δ₀ zeitliches Prüfintervall
Δₓ zeitlicher Grenzabstand
x erster Lichtpuls der zwei benachbarten der Lichtpulse
x+1 zweiter Lichtpuls der zwei benachbarten der Lichtpulse
r Rot
g Grün
b Blau
w Weiß
A späterer Lichtpuls in der einen der zwei der Pulssequenzen
B späterer Lichtpuls in der anderen der zwei der Pulssequenzen
Δ_{A} zeitlicher Abstand zwischen dem ersten Lichtpuls und dem späteren in der einen der zwei der Pulssequenzen
Δ_{B} zeitlicher Abstand zwischen dem ersten Lichtpuls und dem späteren in der anderen der zwei der Pulssequenzen
19 erste Dauer
20 zweite Dauer
21 Pupillendurchmesserverlauf während der einen der zwei der Pulssequenzen
22 Pupillendurchmesserverlauf während der anderen der zwei der Pulssequenzen

## Patentansprüche

1. Pupillometer mit einer Lichtquelle, die eingerichtet ist, einen Menschen mit einer Pulssequenz zu beleuchten, so dass ein Pupillenreflex bei dem Menschen hervorgerufen wird, einer Kamera, die eingerichtet ist, eine Bildsequenz mit Bildern eines Auges des Menschen aufzunehmen, das eine Pupille mit dem Pupillenreflex zeigt, und einer Signalauswerteeinheit, die eingerichtet ist, in den Bildern einen Pupillendurchmesser und daraus einen Pupillendurchmesserverlauf (12, 13) zu bestimmen, wobei die Pulssequenz eine Mehrzahl von zeitlich aufeinanderfolgenden Lichtpulsen (1 bis 11) aufweist und zeitliche Abstände (Δ₁ bis Δ₁₀) zwischen zwei benachbarten der Lichtpulse derart kurz sind, dass der Pupillendurchmesser während der Pulssequenz sich nicht auf einen Ausgangspupillendurchmesser (16) erholt, der am Zeitpunkt des ersten Lichtpulses (1) der Pulssequenz vorliegt, und bei der mindestens zwei der Lichtpulse eine unterschiedliche Farbe haben,
**dadurch gekennzeichnet, dass**
die Lichtpulse (1 bis 11) in der Pulssequenz zeitlich derart angeordnet sind, dass die zeitlichen Abstände (Δ₁ bis Δ₁₀) in Richtung fortschreitender Zeit immer kürzer werden, dass
das Pupillometer eingerichtet ist, einen zeitlichen Grenzabstand (Δₓ) zwischen zwei benachbarten der Lichtpulse (x, x+1) zu bestimmen, bei dem die Pupille ihren Pupillendurchmesser nicht mehr verändert,
dass das Pupillometer eingerichtet ist, den Grenzabstand (Δₓ) zu bestimmen, indem in der Richtung fortschreitender Zeit für jeweils zwei benachbarte der Lichtpulse (x, x+1) in dem Pupillendurchmesserverlauf (12, 13) geprüft wird, ob der Pupillendurchmesser in einem zeitlichen Prüfintervall (Δ₀) innerhalb eines Toleranzbereichs (18) liegt, wobei der Toleranzbereich (18) maximal 10 % des Ausgangspupillendurchmessers (16) beträgt.

2. Pupillometer mit einer Lichtquelle, die eingerichtet ist, einen Menschen mit Pulssequenzen zu beleuchten, so dass jeweils ein Pupillenreflex bei dem Menschen hervorgerufen wird, einer Kamera, die eingerichtet ist, eine Bildsequenz mit Bildern eines Auges des Menschen aufzunehmen, das eine Pupille mit dem Pupillenreflex zeigt, und einer Signalauswerteeinheit, die eingerichtet ist, in den Bildern einen Pupillendurchmesser und daraus einen Pupillendurchmesserverlauf (12, 13) zu bestimmen, wobei die Pulssequenzen jeweils eine Mehrzahl von zeitlich aufeinanderfolgenden Lichtpulsen (1 bis 11) aufweisen und zeitliche Abstände (Δ₁ bis Δ₁₀) zwischen zwei benachbarten der Lichtpulse derart kurz sind, dass der Pupillendurchmesser während der jeweiligen Pulssequenz sich nicht auf einen Ausgangspupillendurchmesser (16) erholt, der am Zeitpunkt des ersten Lichtpulses (1) der jeweiligen Pulssequenz vorliegt, wobei die Lichtquelle eingerichtet ist, den Menschen mit zwei der Pulssequenzen zu beleuchten, wobei der zeitliche Pulssequenzabstand zwischen den zwei Pulssequenzen derart lang ist, dass sich der Pupillendurchmesser auf den Ausgangspupillendurchmesser (16) erholt, wobei in einer der zwei Pulssequenzen ein erster zeitlicher Abstand (Δ_{A}) zwischen dem ersten Lichtpuls (1) und einem späteren Lichtpuls (A) derart ist, dass der spätere Lichtpuls (A) während einer Pupillenkontraktion erfolgt, und in der anderen der zwei Pulssequenzen ein zweiter zeitlicher Abstand (Δ_{B}) zwischen dem ersten Lichtpuls (1) und einem späteren Lichtpuls (B), der dem späteren Lichtpuls (A) in der einen der zwei Pulssequenzen entspricht, derart ist, dass der spätere Lichtpuls (B) während einer Pupillendilatation erfolgt, wobei das Pupillometer eingerichtet ist, eine erste Anfangsbeschleunigung in dem Pupillendurchmesserverlauf (21) nach dem späteren Lichtpuls (A) der einen der zwei Pulssequenzen und eine zweite Anfangsbeschleunigung in dem Pupillendurchmesserverlauf (22) nach dem späteren Lichtpuls (B) der anderen der zwei Pulssequenzen zu bestimmen.

3. Pupillometer gemäß Anspruch 2, wobei in jeder der zwei Pulssequenzen mindestens zwei der Lichtpulse eine unterschiedliche Farbe haben.

4. Pupillometer gemäß Anspruch 2 oder 3, wobei die Lichtpulse (1 bis 11) in jeder Pulssequenz zeitlich derart angeordnet sind, dass die zeitlichen Abstände (Δ₁ bis Δ₁₀) in Richtung fortschreitender Zeit immer kürzer werden.

5. Pupillometer gemäß Anspruch 4, wobei das Pupillometer eingerichtet ist, einen zeitlichen Grenzabstand (Δₓ) zwischen zwei benachbarten der Lichtpulse (x, x+1) zu bestimmen, bei dem die Pupille ihren Pupillendurchmesser nicht mehr verändert.

6. Pupillometer gemäß Anspruch 5, wobei das Pupillometer eingerichtet ist, den Grenzabstand (Δₓ) zu bestimmen, indem in der Richtung fortschreitender Zeit für jeweils zwei benachbarte der Lichtpulse (x, x+1) in dem Pupillendurchmesserverlauf (12, 13) geprüft wird, ob der Pupillendurchmesser in einem zeitlichen Prüfintervall (Δ₀) innerhalb eines Toleranzbereichs (18) liegt, insbesondere wobei der Toleranzbereich (18) maximal 10 %, insbesondere maximal 5 %, des Ausgangspupillendurchmessers (16) beträgt.

7. Pupillometer gemäß Anspruch 1, wobei der Toleranzbereich (18) maximal 5 % des Ausgangspupillendurchmessers (16) beträgt.

8. Pupillometer gemäß Anspruch 1, 6 oder 7, wobei das zeitliche Prüfintervall (Δ₀) mindestens so lang wie der zeitliche Abstand zwischen den jeweiligen zwei benachbarten der Lichtpulse (x, x+1) ist und zeitlich nach mindestens einem der jeweiligen zwei der benachbarten Lichtpulse liegt (x, x+1).

9. Pupillometer gemäß einem der Ansprüche 1 bis 8, wobei die zeitlichen Abstände (Δ₁ bis Δ₁₀) maximal 1,00 s sind, und insbesondere minimal 0,10 s sind.

10. Pupillometer gemäß einem der Ansprüche 1 bis 9, wobei die Lichtquelle eingerichtet ist, lediglich eines der Augen des Menschen mit der Pulssequenz zu beleuchten, und die Kamera eingerichtet ist, die Bildsequenz mit den Bildern des anderen Auges des Menschen aufzunehmen, insbesondere ist das Pupillometer eingerichtet, jeweils eine Bildsequenz mit Bildern von jedem der Augen des Menschen aufzunehmen, und insbesondere ist die Signalauswerteeinheit eingerichtet, für jedes der Augen einen jeweiligen Pupillendurchmesserverlauf (12, 13) zu bestimmen.

11. Pupillometer gemäß einem der Ansprüche 1 bis 10, wobei die unterschiedlichen Farben Rot (r), Grün (g), Blau (b) und insbesondere Weiß (w) aufweisen.

12. Pupillometer gemäß einem der Ansprüche 1 bis 11, wobei das Pupillometer eine weitere Lichtquelle aufweist, die eingerichtet ist, das Auge vor jeder der Pulssequenzen mit Dauerlicht mit einer definierten Lichtstärke von sichtbarem Licht zu beleuchten, insbesondere ist die definierte Lichtstärke ausgewählt aus einem Bereich von 5 bis 20 lux.

13. Verfahren zum Betreiben eines Pupillometers gemäß einem der Ansprüche 1 bis 12, mit den Schritten:
a) Beleuchten des Menschen mit einer Lichtquelle, so dass ein Pupillenreflex bei dem Menschen hervorgerufen wird, mit einer Pulssequenz, die eine Mehrzahl von zeitlich aufeinanderfolgenden Lichtpulsen (1 bis 11) aufweist, wobei zeitliche Abstände (Δ₁ bis Δ₁₀) zwischen zwei benachbarten der Lichtpulse derart kurz sind, dass der Pupillendurchmesser während der Pulssequenz sich nicht auf einen Ausgangspupillendurchmesser (16) erholt, der am Zeitpunkt des ersten Lichtpulses (1) der Pulssequenz vorliegt, und bei der mindestens zwei der Lichtpulse eine unterschiedliche Farbe haben;
b) Aufnehmen einer Bildsequenz mit Bildern eines Auges des Menschen mit der Kamera, wobei die Pupille des Auges den Pupillenreflex zeigt;
c) Bestimmen in den Bildern einen Pupillendurchmesser und daraus einen Pupillendurchmesserverlauf (12, 13),
**dadurch gekennzeichnet, dass**
in Schritt a) die Lichtpulse (1 bis 11) in der Pulssequenz zeitlich derart angeordnet werden, dass die zeitlichen Abstände (Δ₁ bis Δ₁₀) in Richtung fortschreitender Zeit immer kürzer werden;
und das Verfahren den Schritt aufweist:
d) Bestimmen eines zeitlichen Grenzabstands (Δₓ) zwischen zwei benachbarten der Lichtpulse (x, x+1), bei dem die Pupille ihren Pupillendurchmesser nicht mehr verändert,
wobei in Schritt d) in der Richtung fortschreitender Zeit für jeweils zwei benachbarte der aufeinanderfolgenden Lichtpulse (x, x+1) in dem Pupillendurchmesserverlauf (12, 13) geprüft wird, ob der Pupillendurchmesser in einem zeitlichen Prüfintervall (Δ₀) innerhalb eines Toleranzbereichs (18) liegt, wobei der Toleranzbereich (18) maximal 10 % des Ausgangspupillendurchmessers (16) beträgt.

14. Verfahren gemäß Anspruch 13, mit den Schritten:
e) Speichern des Pupillendurchmesserverlaufs (12, 13);
f) Wiederholen der Schritte a) bis c) und Vergleichen des in Schritt f) bestimmten Pupillendurchmesserverlaufs (12, 13) mit dem in Schritt e) gespeicherten Pupillendurchmesserverlauf (12, 13).

15. Verfahren gemäß Anspruch 13 oder 14, wobei der Toleranzbereich (18) maximal 5 % des Ausgangspupillendurchmessers (16) beträgt.

16. Verfahren gemäß einem der Ansprüche 13 bis 15, wobei das zeitliche Prüfintervall (Δ₀) mindestens so lang wie der zeitliche Abstand zwischen den jeweiligen zwei benachbarten der Lichtpulse (x, x+1) ist und zeitlich nach mindestens einem der jeweiligen zwei der benachbarten Lichtpulse (x, x+1) liegt.

17. Verfahren gemäß einem der Ansprüche 13 bis 16, wobei die zeitlichen Abstände maximal 1,00 s sind, und insbesondere minimal 0,10 s sind.

18. Verfahren gemäß einem der Ansprüche 13 bis 17, wobei in Schritt a) lediglich eines der Augen des Menschen mit der Pulssequenz beleuchtet wird, und in Schritt b) die Bildsequenz mit den Bildern des anderen Auges des Menschen aufgenommen wird, insbesondere wird jeweils eine Bildsequenz mit Bildern von jedem der Augen des Menschen aufgenommen, und insbesondere wird in Schritt c) für jedes der Augen ein jeweiliger Pupillendurchmesserverlauf (12, 13) bestimmt.

19. Verfahren gemäß einem der Ansprüche 13 bis 18, wobei die unterschiedlichen Farben Rot (r), Grün (g), Blau (b) und insbesondere Weiß (w) aufweisen.

## Claims

1. A pupilometer comprising a light source, which is configured to illuminate a human with a pulse sequence so that a pupil reflex is caused in the human, a camera, which is configured to record an image sequence having images of an eye of the human, which eye exhibits a pupil with the pupil reflex, and a signal evaluation unit, which is configured to determine in the images a pupil diameter and therefrom a pupil diameter curve (12, 13), the pulse sequence having a plurality of temporally successive light pulses (1 to 11), and temporal intervals (Δ₁ to Δ₁₀) between two adjacent light pulses being short such that, during the pulse sequence, the pupil diameter does not recover to an initial pupil diameter (16) which is present at the time of the first light pulse (1) of the pulse sequence, and in which at least two of the light pulses have a different color, **characterized in that** the light pulses (1 to 11) in the pulse sequence are arranged temporally such that the temporal intervals (Δ₁ to Δ₁₀) become increasingly shorter as time progresses, **in that** the pupilometer is configured to determine a temporal limit interval (Δₓ) between two adjacent light pulses (x, x+1), in which the pupil no longer changes its pupil diameter, **in that** the pupilometer is configured to determine the limit interval (Δₓ) by checking, as time progresses, for two adjacent light pulses (x, x+1) in each case in the pupil diameter curve (12, 13), whether the pupil diameter in a temporal checking interval (Δ₀) is within a tolerance range (18), the tolerance range (18) being at most 10% of the initial pupil diameter (16).

2. The pupilometer comprising a light source, which is configured to illuminate a human with pulse sequences so that a pupil reflex is caused in the human, a camera, which is configured to record an image sequence having images of an eye of the human, which eye exhibits a pupil with the pupil reflex, and a signal evaluation unit, which is configured to determine in the images a pupil diameter and therefrom a pupil diameter curve (12, 13), wherein the pulse sequences each have a plurality of temporally successive light pulses (1 to 11), and temporal intervals (Δ₁ to Δ₁₀) between two adjacent light pulses are short such that, during the relevant pulse sequence, the pupil diameter does not recover to an initial pupil diameter (16) which is present at the time of the first light pulse (1) of the relevant pulse sequence, wherein the light source is configured to illuminate the human with two of the pulse sequences, wherein the temporal pulse sequence interval between the two pulse sequences is long enough for the pupil diameter to recover to the initial pupil diameter (16), wherein in one of the two pulse sequences, a first temporal interval (Δ_{A}) between the first light pulse (1) and a later light pulse (A) is such that the later light pulse (A) takes place during a pupil contraction, and in the other of the two pulse sequences a second temporal interval (Δ_{B}) between the first light pulse (1) and a later light pulse (B), which corresponds to the later light pulse (A) in one of the two pulse sequences, is such that the later light pulse (B) takes place during a pupil dilation, wherein the pupilometer is configured to determine a first initial acceleration in the pupil diameter curve (21) after the later light pulse (A) of one of the two pulse sequences and a second initial acceleration in the pupil diameter curve (22) after the later light pulse (B) of the other of the two pulse sequences.

3. The pupilometer according to claim 2, wherein at least two of the light pulses have a different color in each of the two pulse sequences.

4. The pupilometer according to claim 2 or 3, wherein the light pulses (1 to 11) in each pulse sequence are arranged temporally in such a way that the temporal intervals (Δ₁ to Δ₁₀) become increasingly shorter as time progresses.

5. The pupilometer according to claim 4, wherein the pupilometer is configured to determine a temporal limit interval (Δₓ) between two adjacent light pulses (x, x+1), in which the pupil no longer changes its pupil diameter.

6. The pupilometer according to claim 5, wherein the pupilometer is configured to determine the limit interval (Δₓ) by checking, as time progresses, for two adjacent light pulses (x, x+1) in each case in the pupil diameter curve (12, 13), whether the pupil diameter in a temporal checking interval (Δ₀) is within a tolerance range (18), in particular wherein the tolerance range (18) is at most 10%, in particular at most 5%, of the initial pupil diameter (16).

7. The pupilometer according to claim 1, wherein the tolerance range (18) is at most 5% of the initial pupil diameter (16).

8. The pupilometer according to claim 1, 6 or 7, wherein the temporal checking interval (Δ₀) is at least as long as the temporal interval between the relevant two adjacent light pulses (x, x+1) and is temporally after at least one of the relevant two of the adjacent light pulses (x, x+1).

9. The pupilometer according to any of claims 1 to 8, wherein the temporal intervals (Δ₁ to Δ₁₀) are at most 1.00 s, and in particular at least 0.10 s.

10. The pupilometer according to any of claims 1 to 9, wherein the light source is configured to illuminate only one of the eyes of the human with the pulse sequence, and the camera is configured to record the image sequence having the images of the other eye of the human, in particular the pupilometer is configured to record in each case an image sequence having images of each of the eyes of the human, and in particular the signal evaluation unit is configured to determine a relevant pupil diameter curve (12, 13) for each of the eyes.

11. The pupilometer according to any of claims 1 to 10, wherein the different colors show red (r), green (g), blue (b) and in particular white (w).

12. The pupilometer according to any of claims 1 to 11, wherein the pupilometer has a further light source which is configured to illuminate the eye before each of the pulse sequences with continuous light having a defined light intensity of visible light, in particular the defined light intensity is selected from a range of from 5 to 20 lux.

13. A method for operating a pupilometer according to any of claims 1 to 12, comprising the steps of:
a) illuminating the human with a light source, so that a pupil reflex is caused in the human, with a pulse sequence which has a plurality of temporally successive light pulses (1 to 11), temporal intervals (Δ₁ to Δ₁₀) between two adjacent light pulses being short such that, during the pulse sequence, the pupil diameter does not recover to an initial pupil diameter (16) which is present at the time of the first light pulse (1) of the pulse sequence, and in which at least two of the light pulses have a different color;
b) recording an image sequence having images of an eye of the human with the camera, the pupil of the eye exhibiting the pupil reflex;
c) determining in the images a pupil diameter and therefrom a pupil diameter curve (12, 13),
**characterized in that** in step a), the light pulses (1 to 11) in the pulse sequence are arranged temporally such that the temporal intervals (Δ₁ to Δ₁₀) become increasingly shorter as time progresses;
and the method comprises the step of:
d) determining a temporal limit interval (Δₓ) between two adjacent light pulses (x, x+1), in which the pupil no longer changes its pupil diameter,
in step d) it being checked, as time progresses, for two adjacent of the successive light pulses (x, x+1) in each case in the pupil diameter curve (12, 13), whether the pupil diameter is in a temporal checking interval (Δ₀) within a tolerance range (18), the tolerance range (18) being at most 10% of the initial pupil diameter (16).

14. The method according to claim 13, comprising the steps of:
e) storing the pupil diameter curve (12, 13);
f) repeating steps a) to c) and comparing the pupil diameter curve (12, 13) determined in step f) with the pupil diameter curve (12, 13) stored in step e).

15. The method according to claim 13 or 14, wherein the tolerance range (18) is at most 5% of the initial pupil diameter (16).

16. The method according to any of claims 13 to 15, wherein the temporal checking interval (Δ₀) is at least as long as the temporal interval between the relevant two adjacent light pulses (x, x+1) and is temporally after at least one of the relevant two of the adjacent light pulses (x, x+1).

17. The method according to any of claims 13 to 16, wherein the temporal intervals are at most 1.00 s, and in particular at least 0.10 s.

18. The method according to any of claims 13 to 17, wherein in step a) only one of the eyes of the human is illuminated with the pulse sequence, and in step b) the image sequence having the images of the other eye of the human is recorded, in particular an image sequence having images of each of the eyes of the human is recorded in each case, and in particular in step c) a relevant pupil diameter curve (12, 13) is determined for each of the eyes.

19. The method according to any of claims 13 to 18, wherein the different colors show red (r), green (g), blue (b) and in particular white (w).

## Revendications

1. Pupillomètre comprenant une source de lumière agencée pour éclairer une personne avec une séquence d'impulsions de manière à évoquer un réflexe pupillaire dans la personne, une caméra agencée pour enregistrer une séquence d'images avec des images d'un oeil d'une personne présentant une pupille avec le réflexe pupillaire et une unité d'évaluation de signal agencée pour déterminer dans les images un diamètre pupillaire et de là une courbe du diamètre pupillaire (12, 13), dans lequel la séquence des impulsions a une pluralité d'impulsions lumineuses successives dans le temps (1 à 11) et les intervalles de temps (Δ₁ à Δ₁₀) entre deux impulsions lumineuses adjacentes sont si courts que le diamètre pupillaire pendant la séquence des impulsions ne revient pas au diamètre pupillaire initial (16) qui est présent au moment de la première impulsion lumineuse (1) de la séquence d'impulsions et dans lequel au moins deux des impulsions lumineuses ont une couleur différente,
**caractérisé en ce que**
les impulsions lumineuses (1 à 11) dans la séquence d'impulsions sont disposées dans le temps de telle sorte que les intervalles de temps (Δ₁ à Δ₁₀) deviennent de plus en plus courts dans le sens de progression du temps,
**en ce que** le pupillomètre est agencé pour déterminer une distance limite (Δₓ) entre deux impulsions lumineuses adjacentes (x, x+1), à laquelle la pupille ne change plus son diamètre pupillaire,
**en ce que** le pupillomètre est agencé pour déterminer la distance limite (Δₓ), dès lors dans le sens de progression du temps l'on vérifie, pour deux impulsions lumineuses voisines (x, x+1) dans la courbe du diamètre pupillaire (12, 13), si le diamètre pupillaire se trouve dans un intervalle de temps de test (Δ₀) à l'intérieur d'un intervalle de tolérance (18), l'intervalle de tolérance (18) étant 10% au maximum du diamètre pupillaire initial (16).

2. Pupillomètre comprenant une source de lumière agencée pour éclairer une personne avec des séquences d'impulsions de manière à évoquer un réflexe pupillaire dans la personne, une caméra agencée pour enregistrer une séquence d'images avec des images d'un oeil d'une personne présentant une pupille avec le réflexe pupillaire et une unité d'évaluation de signal agencée pour déterminer dans les images un diamètre pupillaire et de là un profil du diamètre pupillaire (12, 13), dans lequel les séquences d'impulsions ont chacune une pluralité d'impulsions lumineuses successives dans le temps (1 à 11) et les intervalles de temps (Δ₁ à Δ₁₀) entre deux impulsions lumineuses adjacentes sont si courts que le diamètre pupillaire pendant la séquence d'impulsions respective ne revient pas au diamètre pupillaire initial (16) qui est présent au moment de la première impulsion lumineuse (1) de la séquence d'impulsions respective, la source lumineuse étant agencée pour éclairer la personne avec deux séquences d'impulsions, l'intervalle de temps de la séquence d'impulsions entre les deux séquences d'impulsions étant si long que le diamètre pupillaire revient au diamètre pupillaire initial (16), dans lequel, dans l'une des deux séquences d'impulsions, un premier intervalle de temps (Δ_{A}) entre la première impulsion lumineuse (1) et une impulsion lumineuse successive (A) est tel que l'impulsion lumineuse successive (A) se produit pendant une contraction de la pupille, et dans l'autre des deux séquences d'impulsions un deuxième intervalle de temps (Δ_{B}) entre la première impulsion lumineuse (1) et une impulsion lumineuse successive (B), correspondant à l'impulsion lumineuse successive (A) dans l'une des deux séquences d'impulsions, est tel que l'impulsion lumineuse successive (B) se produit pendant une dilatation de la pupille, le pupillomètre étant agencé pour déterminer une première accélération initiale dans la courbe du diamètre pupillaire (21) après l'impulsion lumineuse successive (A) de l'une des deux séquences d'impulsions et une deuxième accélération initiale dans la courbe du diamètre pupillaire (22) après l'impulsion lumineuse successive (B) de l'autre des deux séquences d'impulsions.

3. Pupillomètre selon la revendication 2, dans lequel dans chacune des deux séquences d'impulsions au moins deux des impulsions lumineuses ont une couleur différente.

4. Pupillomètre selon la revendication 2 ou 3, dans lequel les impulsions lumineuses (1 à 11) dans chaque séquence d'impulsions sont disposées dans le temps de telle sorte que les intervalles de temps (Δ₁ à Δ₁₀) deviennent de plus en plus courts dans le sens de progression du temps.

5. Pupillomètre selon la revendication 4, dans lequel le pupillomètre est agencé pour déterminer une distance limite (Δₓ) dans le temps entre deux impulsions lumineuses adjacentes (x, x+1), à laquelle la pupille ne change plus son diamètre pupillaire.

6. Pupillomètre selon la revendication 5, dans lequel le pupillomètre est agencé pour déterminer la distance limite (Δₓ) en contrôlant dans le sens de progression du temps pour chaque deux impulsions lumineuses adjacentes (x, x+1) dans la courbe du diamètre pupillaire (12, 13) si le diamètre pupillaire dans un intervalle de temps de test (Δ₀), se trouve dans un intervalle de tolérance (18), l'intervalle de tolérance (18) étant notamment 10% au maximum, notamment 5% au maximum, du diamètre pupillaire initial (16).

7. Pupillomètre selon la revendication 1, dans lequel l'intervalle de tolérance (18) est égal à 5% au maximum du diamètre pupillaire initial (16).

8. Pupillomètre selon la revendication 1, 6 ou 7, dans lequel l'intervalle de temps de test (Δ₀) est au moins aussi long que la distance dans le temps entre les deux impulsions lumineuses adjacentes (x, x+1) respectives et postérieur dans le temps au moins à l'une des deux impulsions lumineuses adjacentes (x, x+1) respectives.

9. Pupillomètre selon l'une des revendications 1 à 8, dans lequel les intervalles de temps (Δ₁ à Δ₁₀) sont de 1,00 s au maximum, notamment d'au moins 0,10 s.

10. Pupillomètre selon l'une des revendications 1 à 9, dans lequel la source lumineuse est agencée pour n'éclairer que l'un des yeux de la personne avec la séquence d'impulsions, et la caméra est agencée pour enregistrer la séquence d'images avec les images de l'autre oeil de la personne, notamment le pupillomètre est agencé pour enregistrer une séquence d'images avec des images de chacun des yeux de la personne, et notamment l'unité d'évaluation de signal est agencée pour déterminer pour chacun des yeux une courbe du diamètre pupillaire (12, 13) respective.

11. Pupillomètre selon l'une des revendications 1 à 10, dans lequel les couleurs différentes sont le rouge (r), le vert (g), le bleu (b) et notamment le blanc (w).

12. Pupillomètre selon l'une des revendications 1 à 11, dans lequel le pupillomètre présente une source de lumière supplémentaire qui est agencée pour éclairer l'œil avant chacune des séquences d'impulsions avec une lumière continue avec une intensité lumineuse définie de lumière visible, notamment l'intensité lumineuse définie est sélectionnée dans un intervalle compris entre 5 et 20 lux.

13. Méthode de fonctionnement d'un pupillomètre selon l'une des revendications 1 à 12, comprenant les étapes de:
a) éclairer la personne avec une source lumineuse de manière à évoquer un réflexe pupillaire dans la personne, avec une séquence d'impulsions qui a une pluralité d'impulsions lumineuses successives dans le temps (1 à 11), dans laquelle les intervalles de temps (Δ₁ à Δ₁₀) entre deux impulsions lumineuses adjacentes sont si courts que le diamètre pupillaire pendant la séquence d'impulsions ne revient pas au diamètre pupillaire initial (16) qui est présent au moment de la première impulsion lumineuse (1) de la séquence d'impulsions, et dans laquelle au moins deux des impulsions lumineuses ont une couleur différente,
b) enregistrer avec la caméra une séquence d'images avec des images d'un oeil de la personne, dans lesquelles la pupille de l'œil montre le réflexe pupillaire;
c) déterminer un diamètre pupillaire dans les images et de là une courbe du diamètre pupillaire (12, 13),
**caractérisée en ce que**
à l'étape a), les impulsions lumineuses (1 à 11) dans la séquence d'impulsions sont disposées dans le temps de telle sorte que les intervalles de temps (Δ₁ à Δ₁₀) deviennent de plus en plus courts dans le sens de progression du temps;
et la méthode comprend l'étape de:
d) déterminer une distance limite (Δₓ) dans le temps entre deux impulsions lumineuses adjacentes (x, x+1) à laquelle la pupille ne change plus son diamètre pupillaire,
dans laquelle, à l'étape d), dans le sens de progression du temps, l'on vérifie pour chaque deux impulsions lumineuses (x, x+1) adjacentes successives dans la courbe du diamètre pupillaire (12, 13) si le diamètre pupillaire dans un intervalle de temps de test (Δ₀) se trouve à l'intérieur d'un intervalle de tolérance (18), l'intervalle de tolérance (18) correspondant à 10% au maximum du diamètre pupillaire initial (16).

14. Méthode selon la revendication 13, comprenant les étapes de:
e) sauvegarder la courbe du diamètre pupillaire (12, 13);
f) répéter les étapes a) à c) et comparer la courbe du diamètre pupillaire (12, 13) déterminée à l'étape f) avec la courbe du diamètre pupillaire (12, 13) sauvegardée à l'étape e).

15. Méthode selon la revendication 13 ou 14, dans laquelle l'intervalle de tolérance (18) est égal au maximum à 5% du diamètre pupillaire initial (16).

16. Méthode selon l'une des revendications 13 à 15, dans laquelle l'intervalle de temps de test (Δ₀) est au moins aussi long que la distance dans le temps entre les deux impulsions lumineuses adjacentes (x, x+1) respectives et postérieur dans le temps au moins à l'une des deux impulsions lumineuses adjacentes (x, x+1) respectives.

17. Méthode selon l'une des revendications 13 à 16, dans laquelle les intervalles de temps sont de 1,00 s au maximum, et notamment d'au moins 0,10 s.

18. Méthode selon l'une des revendications 13 à 17, dans laquelle à l'étape a) seulement l'un des yeux de la personne est éclairé avec la séquence d'impulsions et à l'étape b) la séquence d'images est enregistrée avec les images de l'autre oeil de la personne, notamment l'on enregistre à chaque fois une séquence d'images avec des images de chacun des yeux de la personne et notamment à la phase c) pour chacun des yeux, l'on détermine une courbe du diamètre pupillaire (12, 13) respective.

19. Méthode selon l'une des revendications 13 à 18, dans laquelle lesdites couleurs différentes comprennent le rouge (r), le vert (g), le bleu (b) et notamment le blanc (w).
